# FASCICULE DE BREVET EUROPEEN

(11) **EP 1 137 653 B1**
(45) Date de publication et mention de la délivrance du brevet: **15.09.2004**
(21) Numéro de dépôt: 99958302.4
(22) Date de dépôt: 10.12.1999
(51) Int. Cl.: C07H 17/07, A61K 31/70

(54) **TROXERUTINE A FORTE TENEUR EN TRIHYDROXY-ETHYL-RUTOSIDE ET PROCEDE DE PREPARATION**
TROXERUTIN MIT HOHEM TRIHYDROXY-ETHYL-RUTOSIDEGEHALT UND VERFAHREN ZUR HERSTELLUNG
TROXERUTIN WITH HIGH TRIHYDROXY-ETHYL-RUTIN CONTENT AND METHOD FOR PREPARING SAME

(30) Priorité: 11.12.1998 FR 9815710
(43) Date de publication de la demande: 04.10.2001
(73) Titulaire: Laboratoires Negma, 78117 Toussus-Le-Noble (FR)
(72) Inventeur: ESTANOVE, Cyril, F-92100 Boulogne (FR); PRUVOST, François, F-29000 Quimper (FR)
(74) Mandataire: L'Helgoualch, Jean
(86) Numéro de dépôt international: PCT/FR1999/003100
(87) Numéro de publication internationale: WO 2000/035933

(56) Documents cités:
- FR-A- 2 267 327
- CHEMICAL ABSTRACTS, vol. 113, no. 17, 22 octobre 1990 (1990-10-22) Columbus, Ohio, US; abstract no. 152961d, H.CAI ET AL.: "Purification of (hydroxyethyl)rutin Derivatives." page 832; colonne 2; XP002112969 & CN 1 032 658 A (YANTAI PEOPLE'S PHARMACEUTICAL FACTORY)

## Description

La présente invention concerne une préparation à forte teneur en 7,3',4'-trihydroxyéthyl-rutoside à mouillabilité élevée, une composition pharmaceutique à base de troxérutine enrichie en 7,3',4'-trihydroxyéthyl-rutoside, possédant une solubilité améliorée, ainsi qu'un procédé permettant de les préparer.

Le 7,3',4'-trihydroxyéthyl-rutoside est le principal constituant de la troxérutine, qui est une composition utilisée en thérapeutique pour le traitement des symptômes en rapport avec les insuffisances veino-lymphatiques (jambes lourdes, douleur, impatience du primo décubitus) et le traitement des signes fonctionnels liés à la crise hémorroïdaire. La troxérutine est constituée d'un mélange de plusieurs dérivés de la classe des flavonoïdes, et plus précisément des dérivés hydroxyéthylés de rutoside (ou rutine). Les indications thérapeutiques précitées reposent sur les propriétés veinotoniques et vasculoprotectrices de la troxérutine. Les études réalisées chez l'animal et chez l'homme ont montré en effet que les hydroxyéthylrutosides augmentent le tonus veineux et réduisent la perméabilité capillaire.

Le brevet FR-5072 M décrit une troxérutine comprenant un mélange de tri-hydroxyéthyl-rutoside et de tétra-hydroxyéthylrutoside présentant des propriétés de renforcement de la résistance capillaire utiles pour le traitement de maladies causées par les troubles de la microcirculation sanguine. Cette troxérutine est préparée à partir de rutoside et de carbonate d'éthylène, à chaud en présence d'un catalyseur alcalin. Le brevet US-A-3.420.815 décrit un procédé similaire consistant à faire réagir l'oxyde d'éthylène sur la rutine en présence d'une base, puis à effectuer une cristallisation. Selon ce brevet, la réaction progresse en faisant apparaître successivement les dérivés mono-, di-, tri- et tétra-hydroxyéthylés de la rutine qui sont isolés après distillation sous pression réduite et séchage. Une variante de ce procédé décrite dans le brevet FR-A-2.267.327 permet de préparer le 7-mono-β-hydroxyéthyl-rutoside, qui présente une activité comparable de régulation de la perméabilité capillaire et de renforcement de la résistance capillaire, par réaction d'oxyde d'éthylène et de rutoside en présence d'un agent complexant.

La troxérutine préparée par les procédés usuels de la technique et couramment utilisée aujourd'hui en thérapeutique comprend généralement environ 80 à 85 % de dérivé trihydroxyéthylé (7,3',4'-trihydroxyéthyl-rutoside), 8 à 10 % environ de dérivé tétrahydroxyéthylé (5,7,3',4'-tétrahydroxyéthyl-rutoside), et 4 à 5 % de dérivé dihydroxyéthylé (7,4'-dihydroxyéthyl-rutoside), le reste étant constitué par les dérivés 7,3',4'- et 7,5',4'-trihydroxyéthylisoquercitrine-3-glucoside et 7,4'-dihydroxyéthylkaempférol-3-rutinoside. La troxérutine préparée par les techniques usuelles se mouille difficilement dans l'eau, ce qui entraîne un temps de dissolution important et constitue un inconvénient pour la préparation de formes pharmaceutiques administrables sous forme de poudre pour solutions, et pour la préparation extemporanée rapide desdites solutions par les utilisateurs auxquelles ces formes pharmaceutiques sont destinées.

Les travaux entrepris par la demanderesse ont permis de montrer qu'il est possible de préparer une troxérutine présentant une teneur élevée en 7,3',4'-trihydroxyéthyl-rutoside, et que cette préparation (ci-après dénommée "troxérutine enrichie" en raison de sa forte teneur en 7,3',4'-trihydroxyéthyl-rutoside) représente le meilleur compromis entre les propriétés physico-chimiques (mouillabilité dans l'eau) et l'activité pharmacodynamique (constantes pharmacocinétiques, y compris les liaisons aux protéines plasmatiques, et propriétés rhéologiques).

La présente invention a donc plus particulièrement pour objet une troxérutine enrichie ayant une teneur d'au moins 92 % en poids de 7,3',4'-trihydroxyéthyl-rutoside et une mouillabilité exprimée en minutes inférieure à 10 minutes, lorsque cette mouillabilité est mesurée dans un essai consistant à mesurer le temps que mettent 3,5 g d'une poudre de cette troxérutine enrichie à quitter la surface d'un bécher contenant 100 ml d'eau, à une température stabilisée de 20°C, lorsque cette poudre de troxérutine enrichie est versée à la surface de l'eau dans ce bécher, et ayant une mouillabilité inférieure en moyenne à 100 secondes lorsque cette mouillabilité est mesurée dans un essai consistant à mesurer le temps que met cette troxérutine enrichie à être mouillée par l'eau contenue dans un récipient, tel qu'un bécher, lorsque cette troxérutine enrichie a été posée à la surface de l'eau, sous la forme de carottes de taille 2 et de 3 mm de hauteur et pesant 63 ± 2 mg, à une température stabilisée de 20°C.

Le compactage préalable peut être réalisé par tout type de dispositif approprié, par exemple dans un dispositif de production de gélules (géluleuses).

L'invention concerne une troxérutine enrichie comprenant au moins 92% en poids de 7,3',4'-trihydroxyéthyl-rutoside, au plus 5 % en poids de 5,7,3',4'-tétrahydroxyéthyl-rutoside, et au plus 4 % en poids de 7,4'-dihydroxyéthyl-rutoside, et plus particulièrement une troxérutine enrichie du type ci-dessus, qui contient des teneurs réduites de 5,7,3',4'-tétrahydroxyéthyl-rutoside (au plus 5 % en poids), et de 7,4'-dihydroxyéthyl-rutoside (au plus 4 % en poids).

L'invention a aussi pour objet une composition pharmaceutique comprenant à titre de principe actif une troxérutine enrichie contenant au moins 92 % en poids de 7,3',4'-trihydroxyéthyl-rutoside, ce principe actif pouvant également contenir au plus 5 % en poids de 5,7,3',4'-tétrahydroxyéthyl-rutoside, et au plus 4 % en poids de 7,4'-dihydroxyéthyl-rutoside, ces compositions pharmaceutiques présentant d'intéressantes propriétés améliorant leur efficacité pharmaceutique, et possédant de bonnes propriétés de mouillabilité et de solubilité dans l'eau.

En particulier, l'invention concerne des compositions pharmaceutiques ayant une solubilité élevée et une grande vitesse de dissolution, à base de troxérutine enrichie associée à un excipient propice à son administration par voie orale - par exemple un poids égal de mannitol - ayant un temps de dissolution inférieur en moyenne à 140 secondes lorsque ce temps est mesuré dans un essai consistant à verser 7,25 g de grain dans un bécher de 250 ml (diamètre de col 78 mm, hauteur 95 mm) qui contient 200 ml d'eau (température de la pièce aux environs de 20°C, agitation avec un barreau magnétique de 35 x 6.5 mm, vitesse 4 de l'appareil (IKAMAG) et à mesurer le temps de solubilisation du grain.

La présente invention a encore pour objet un nouveau procédé de préparation de troxérutine enrichie à mouillabilité élevée, dans de bonnes conditions de rendement.

Le procédé de préparation conforme à la présente invention consiste à faire réagir à chaud la rutine avec un excès d'oxyde d'éthylène dans de l'eau en présence d'une base, puis à provoquer une cristallisation dans un alcool, et il se distingue en ce que l'on concentre le milieu réactionnel de manière à obtenir, dans le milieu de cristallisation, une teneur en eau inférieure à 8 %, et de préférence comprise entre 1 et 6 %.

Suivant une forme préférentielle de mise en oeuvre du procédé de l'invention, la cristallisation s'effectue dans un alcool choisi parmi le méthanol et l'isopropanol, isolément ou, de préférence, en mélange, à une température de fin de cristallisation comprise entre 35 et 15°C. Il est tout particulièrement avantageux d'effectuer la cristallisation en choisissant un profil de descente en température adapté à la cinétique de cristallisation du dérivé tri-hydroxyéthylé par rapport aux dérivés di- et tétra-hydroxyéthylés de rutoside. En effet, les conditions opératoires suivant le procédé de l'invention, en appliquant une descente en température rapide et des ratios de solvants déterminés, permettent de provoquer la précipitation préférentielle du dérivé trihydroxyéthylé et de limiter la précipitation des dérivés tétra- et di-hydroxyéthylés, la concentration finale dans le milieu étant insuffisante pour rendre possible la cristallisation de ces derniers.

Comme indiqué ci-dessus, la vitesse de descente en température doit être rapide, dans des conditions industrielles de mise en oeuvre, et de préférence supérieure à environ 20°C par heure, et de préférence 30°C par heure.

La base ajoutée dans le milieu réactionnel peut être choisie parmi l'hydroxyde de sodium ou de potassium, ou le carbonate de sodium, de potassium, de lithium ou de calcium.

On obtient ainsi une troxérutine enrichie comprenant au moins 92% en poids de 7,3',4'-trihydroxyéthyl-rutoside, au plus 5 % en poids de 5,7,3',4'-tétrahydroxyéthyl-rutoside, et au plus 4 % en poids de 7,4'-dihydroxyéthyl-rutoside. De préférence, la teneur en 7,4'-dihydroxyéthyl-rutoside est comprise entre 1 et 3% en poids, tandis que la teneur en 5,7,3',4'-tétrahydroxyéthyl-rutoside est comprise entre 2 et 4 % en poids.

D'une manière avantageuse, la troxérutine enrichie de l'invention comprend au moins 93 % de 7,3',4'-trihydroxyéthyl-rutoside, 2 à 3,5 % de 5,7,3',4'-tétrahydroxyéthyl-rutoside et 1,7 à 2,5 % de 7,4'-dihydroxyéthyl-rutoside. Comme indiqué ci-dessus, elle peut contenir quelques traces de dérivés 7,3',4'-et 7,5',4'-trihydroxyéthylisoquercitrine-3-glucoside et 7,4'-dihydroxyéthylkaempférol-3-rutinoside.

La troxérutine enrichie conforme à la présente invention contient au moins 92 % en poids de 7,3',4'-trihydroxyéthyl-rutoside, comme indiqué ci-dessus. Elle peut aussi contenir l'isomère constitué par le 5,7,4'-trihydroxyéthyl-rutoside, qui peut ne pas être distingué facilement de l'isomère ci-dessus par les techniques d'analyse courante. On convient, dans la présente description que la dénomination 7,3',4'-trihydroxyéthyl-rutoside englobe au besoin le deuxième isomère. De même, la dénomination 7,4'-dihydroxyéthyl-rutoside peut englober, le cas échéant, l'isomère 7,3'-dihydroxyéthyl-rutoside.

La troxérutine enrichie préparée suivant l'invention et présentant les teneurs indiquées ci-dessus en dérivés de rutoside di-, tri- et tétra-hydroxyéthylés, possède d'intéressantes propriétés. En particulier la troxérutine enrichie de l'invention présente une bonne mouillabilité dans l'eau et une inhibition de l'agrégation des globules rouges améliorée par comparaison avec les troxérutines usuelles.

En effet, les études ex vivo réalisées sur sang humain ont montré que l'inhibition de l'agrégation des globules rouges était significativement améliorée en présence de tri- ou de tétrahydroxyéthylrutosides et que le dérivé trihydroxyéthylé a une meilleure efficacité que le dérivé tétrahydroxyéthylé. Ce différentiel d'efficacité a été confirmé par la supériorité de la troxérutine enrichie suivant l'invention (teneur en trihydroxyéthylrutoside voisine de 95 %) par rapport aux troxérutines disponibles dans le commerce (teneur voisine de 84 %). L'intérêt d'une diminution de l'agrégation des globules rouges réside dans une réduction de la viscosité du sang total, et par conséquent dans une meilleure fluidité du sang. Cette propriété se manifeste par une augmentation du retour veineux et du débit micro-circulatoire (augmentation de la densité des capillaires perfusés).

La mouillabilité de la troxérutine enrichie préparée suivant l'invention a été comparée avec celle des troxérutines disponibles dans le commerce comprenant environ 84 % de dérivé trihydroxyéthylé, environ 8 % de dérivé tétrahydroxyéthylé, et environ 4 % de dérivé dihydroxyéthylé de rutoside, le reste étant constitué par les dérivés hydroxyéthylés d'isoquercitrine-3-glucoside et de kaempférol-3-rutinoside précités.

On verse 3,5 g de troxérutine enrichie à tester dans un bécher de 250 ml (forme basse) contenant 100 ml d'eau dont la température est stabilisée à celle du laboratoire (aux environs de 20°C). On relève la température au début du test et on mesure le temps nécessaire pour que la troxérutine enrichie soit mouillée en totalité, c'est-à-dire qu'elle tombe en totalité au fond du bécher.

Le tableau ci-après indique le temps nécessaire pour parvenir à une dispersion proche de 100 %, mesuré sur trois échantillons d'une troxérutine enrichie de l'invention et de troxérutines du commerce. Chaque essai a été effectué en maintenant la température de la solution aqueuse à 20°C, sans agitation.

| **Tableau comparatif** | | | |
|---|---|---|---|
| Troxérutine | Temps (min.) | Moyenne (min.) | Ecart-type |
| Invention | 3,5 | | |
| | 4,5 | 5,5 | 2,6 |
| | 8,4 | | |
| Comparaison | 65 | | |
| | 70 | 69,3 | 4,0 |
| | 73 | | |

De même, on dépose de la troxérutine enrichie sous forme d'une carotte de poudre de taille 2 et de 3 mm de hauteur et pesant 63 ± 3 mg à la surface d'un bécher de 80 ml contenant 100 ml d'eau. On relève la température de l'eau au début du test et on mesure le temps nécessaire pour que la troxérutine enrichie soit mouillée en totalité c'est-à-dire qu'elle change de couleur en totalité.

Le tableau ci-après indique le temps moyen nécessaire pour mouiller des carottes de troxérutine enrichie de l'invention et de troxérutine du commerce.

| Troxérutine | Temps moyen (sec) |
|---|---|
| Invention | <100 sec |
| Comparaison | 325 sec |

Les résultats des deux essais ci-dessus montrent que la troxérutine enrichie de l'invention se distingue des troxérutines de référence par une mouillabilité nettement améliorée.

L'étude réalisée sur des compositions pharmaceutiques à base de troxérutine enrichie conforme à la présente invention a montré que les propriétés rhéologiques mises en évidence dépendent essentiellement des fortes doses du 7,3',4'-trihydroxyéthyl-rutoside, c'est-à-dire au moins 92 % ; en effet, des études in vitro ont établi une relation entre les phénomènes de correction rhéologique (amélioration de la cinétique d'agrégation et des constantes de désagrégation des globules rouges) et la concentration en 7,3',4'-trihydroxyéthyl-rutoside.

De plus, on a constaté que la troxérutine enrichie de l'invention est moins liée aux protéines plasmatiques, ce qui limite le risque d'interaction médicamenteuse. Cet avantage est d'autant plus important dans le cas de patients âgés qui peuvent être amenés à prendre plusieurs médicaments.

Les compositions pharmaceutiques à base de troxérutine enrichie présentent une meilleure solubilisation, en particulier dans les conditions suivantes.

On prépare un mélange contenant par exemple : 500 g de la troxérutine enrichie à tester, 500 g de mannitol et 0.035 g de saccharinate de sodium. On mélange les composés et on granule par un mélange éthanol/eau.

On sèche le grain obtenu à l'étuve. Le grain sec est calibré sur une grille dont le diamètre nominal des mailles est de 800 µm, puis on y ajoute 35,71 g d'arôme orange. On mélange pour homogénéiser pendant 5 min. On verse 7,25 g de grain dans un bécher de 250 ml (diamètre de col 78 mm, hauteur 95 mm) qui contient 200 ml d'eau (température de la pièce aux environs de 20°C, agitation avec un barreau magnétique de 35 x 6.5 mm, vitesse 4 de l'appareil (IKAMAG)). Le temps nécessaire pour la solubilisation du grain est mesuré.

| **RESULTATS** | |
|---|---|
| | **Moyenne en secondes** |
| Composition pharmaceutique à base de troxérutine enrichie | < 140 |
| Composition pharmaceutique à base de troxérutine classique | 226 |

On constate l'amélioration importante de la solubilisation du produit fini fabriqué avec la troxérutine enrichie de l'invention. Les compositions pharmaceutiques finales à base de troxérutine enrichie requièrent également des volumes de liquide de mouillage moins importants pour réaliser les opérations nécessaires à la granulation, lorsque le produit doit être finalement présenté sous forme de granulés. De 150 g d'eau pour mouiller 1000 g d'une préparation à base de troxérutine classique et d'un poids semblable de mannitol, on passe à moins de 100 g d'eau pour mouiller la même quantité de la composition selon l'invention à base de troxérutine purifiée.

| **RESULTATS** | |
|---|---|
| | **Liquide de mouillage en g** |
| Composition pharmaceutique à base de troxérutine enrichie | < 100 g |
| Composition pharmaceutique à base de troxérutine classique | 150 g |

Les exemples suivants illustrent plus en détail l'invention sans en limiter la portée. Sauf indication contraire, toutes les parties et pourcentages sont exprimés en poids.

### Exemple 1

100 g de rutine sont traités à chaud, à environ 75°C, par 28 g d'oxyde d'éthylène dans 100 ml d'eau additionnés de 1,1 g de soude, et le mélange réactionnel est maintenu sous agitation pendant environ 6 heures. La réaction est suivie par chromatographie HPLC, et la fin de la réaction est détectée lorsque les proportions relatives de dérivés di-, tri- et tétra-hydroxyéthylés de rutoside sont respectivement de 4 %, 88 % et 7 à 8 %. Le milieu est alors acidifié par addition d'acide sulfurique pour neutraliser l'alcali présent.

Le milieu aqueux est concentré sous pression réduite (environ 2.10⁴ Pa) à une température comprise entre 60 et 70°C, de telle sorte que la concentration en eau soit proche de 2 % dans le milieu de cristallisation final.

Le concentrat est repris dans 240 ml de méthanol, puis filtré pour éliminer les sels formés. On ajoute à la solution 220 ml d'isopropanol, et, après contrôle de la teneur en eau on procède à une cristallisation pendant 8 heures environ, en faisant descendre la température de 65°C à 25°C pendant les deux premières heures et en maintenant la température entre 25 et 20°C pendant les 6 heures suivantes.

On obtient ainsi 89,4 g (rendement 80 %) de troxérutine titrant 92 % de dérivé trisubstitué, 4 % de dérivé tétra-substitué et 3 % de dérivé disubstitué, le reste étant constitué par les dérivés hydroxyéthylés d'isoquercitrine-3-glucoside et de kaempférol-3-rutinoside précités.

### Exemple 2

On procède comme dans l'Exemple 1, mais la cristallisation s'effectue pendant environ 3 à 4 heures, pendant lesquelles on fait diminuer la température de 65°C à 30°C, puis on maintient la température entre 30 et 25°C pendant environ 2 heures.

On obtient ainsi 87,2 g (rendement 78 %) de troxérutine titrant 93 % de dérivé trisubstitué, 3,5 % de dérivé tétra-substitué et 2,5 % de dérivé disubstitué, le reste étant constitué par les mêmes dérivés que ci-dessus.

### Exemple 3

On procède comme dans l'Exemple 1, mais on fait passer la solution aqueuse sur des résines échangeuses d'ion de type cationiques fortes, puis anioniques fortes, avant filtration. Le milieu est ensuite concentré de manière à ramener la teneur en eau à une valeur égale à environ 5,2 % dans le milieu final.

Le concentrat est repris dans 800 ml de méthanol, et on ajoute à la solution 30 ml d'isopropanol. La cristallisation s'effectue comme ci-dessus, pendant environ 2 heures, en maintenant la température entre 25 et 15°C pendant environ 1 heure en fin de cristallisation.

On obtient ainsi 84,2 g (rendement 75 %) de troxérutine titrant 95 % de dérivé trisubstitué, 2,8 % de dérivé tétra-substitué et 1,7 % de dérivé disubstitué, le reste étant constitué par les mêmes dérivés que ci-dessus.

## Revendications

1. Troxérutine enrichie ayant une teneur d'au moins 92 % en poids de 7,3',4'-trihydroxyéthyl-rutoside, entre 2 et 4 % en poids de 5,7,3',4'-tétrahydroxyéthyl-rutoside, et entre 1 et 3 % en poids de 7,4'-dihydroxyéthyl-rutoside, et une mouillabilité exprimée en minutes inférieure à 10 minutes, lorsque cette mouillabilité est mesurée dans un essai consistant à mesurer le temps que mettent 3,5 g d'une poudre de cette troxérutine enrichie à quitter la surface d'un bécher contenant 100 ml d'eau, à une température stabilisée de 20°C, lorsque cette poudre de troxérutine enrichie est versée à la surface de l'eau dans ce bécher, et une mouillabilité inférieure à 100 secondes lorsque cette mouillabilité est mesurée dans un essai consistant à mesurer le temps que met cette troxérutine enrichie à être mouillée par l'eau contenue dans un récipient, tel qu'un bécher, lorsque cette troxérutine enrichie a été posée à la surface de l'eau, sous la forme de carottes de taille 2 et de 3 mm de hauteur et pesant 63 ± 2 mg, à une température stabilisée de 20°C.

2. Troxérutine enrichie **caractérisée en ce qu'**elle comprend au moins 92 % en poids de 7,3',4'-trihydroxyéthyl-rutoside, entre 2 et 4 % en poids de 5,7,3',4'-tétrahydroxyéthyl-rutoside, et entre 1 et 3 % en poids de 7,4'-dihydroxyéthyl-rutoside.

3. Troxérutine enrichie selon la revendication 1 ou 2, **caractérisée en ce que** sa teneur en 7,3',4'-trihydroxyéthyl-rutoside est supérieure ou égale à 93 % en poids.

4. Troxérutine enrichie selon l'une quelconque des revendications 2 et 3, **caractérisée en ce qu'**elle comprend au moins 93 % de 7,3',4'-trihydroxyéthyl-rutoside, 2 à 3,5 % de 5,7,3',4'-tétrahydroxyéthyl-rutoside et de 1,7 à 2,5 % de 7,4'-dihydroxyéthyl-rutoside.

5. Composition pharmaceutique contenant une troxérutine enrichie selon l'une quelconque des revendications 1 à 4 en association avec un excipient pharmaceutique, de préférence pour administration par voie orale.

6. Composition pharmaceutique selon la revendication 5 ayant une solubilité élevée et une grande vitesse de dissolution, à base de troxérutine enrichie associée à un excipient propice à son administration par voie orale ayant un temps de dissolution inférieur en moyenne à 140 secondes lorsque ce temps est mesuré dans un essai consistant à verser 7.25 g de grain dans un bécher de 250 ml (diamètre de col 78 mm, hauteur 95 mm) qui contient 200 ml d'eau (température de la pièce aux environs de 20°C, agitation avec un barreau magnétique de 35 x 6.5 mm, vitesse 4 de l'appareil (IKAMAG)) et à mesurer le temps de solubilisation du grain.

7. Composition selon la revendication6, **caractérisée en ce que** le principe actif, dont fait partie le 7,3',4'-trihydroxyéthyl-rutoside, est constitué par une troxérutine enrichie dans laquelle le 7,3',4'-trihydroxyéthyl-rutoside est à hauteur d'au moins 92 % en poids.

8. Composition selon la revendication7, **caractérisée en ce que** la troxérutine enrichie a une constitution conforme à celle de l'une quelconque des revendications 2 à 4.

9. Utilisation d'une troxérutine enrichie selon l'une quelconque des revendications 1 à 4 pour la production d'un médicament pour le traitement des symptômes en rapport avec les insuffisances veino-lymphatiques (jambes lourdes, douleur, impatience du primo décubitus) et le traitement des signes fonctionnels liés à la crise hémorroïdaire.

10. Procédé de préparation de troxérutine comprenant au moins 92 % en poids de 7,3',4'-trihydroxyéthyl-rutoside, entre 2 et 4 % en poids de 5,7,3',4'-tétrahydroxyéthyl-rutoside, et entre 1 et 3 % en poids de 7,4'-dihydroxyéthyl-rutoside, consistant à faire réagir à chaud la rutine avec un excès d'oxyde d'éthylène dans de l'eau en présence d'une base, puis à provoquer une cristallisation dans un alcool, **caractérisé en ce que** l'on concentre le milieu réactionnel de manière à obtenir, dans le milieu de cristallisation, une teneur en eaucomprise entre 1 et 8%.

11. Procédé selon la revendication 10, **caractérisé en ce que** la teneur en eau est comprise entre 1 et 6 %.

12. Procédé selon l'une quelconque des revendications 10 et 11, **caractérisé en ce que** le solvant de cristallisation est le méthanol ou l'isopropanol, isolément ou en mélange.

13. Procédé selon l'une quelconque des revendications 10 à 12, **caractérisé en ce que** la cristallisation est effectuée avec une vitesse de descente en température supérieure à environ 20°C par heure pendant 1 à 2 heures.

14. Procédé selon l'une quelconque des revendications 10 à 13, **caractérisé en ce que** la cristallisation s'effectue à une température comprise entre 35 et 15°C.

## Patentansprüche

1. Angereichertes Troxerutin mit einem Mindestgehalt von 92 Gew.-% an 7,3',4'-Trihydroxyethylrutosid, zwischen 2 und 4 Gew.-% an 5,7,3',4'-Tetrahydroxyethylrutosid und zwischen 1 und 3 Gew.-% an 7,4'-Dihydroxyethylrutosid und mit einer in Minuten ausgedrückten Benetzbarkeit von unter 10 Minuten, wenn die Benetzbarkeit in einem Versuch gemessen wird, der darin besteht, dass die Zeit gemessen wird, die 3,5 g eines Pulvers dieses angereichteren Troxerutins benötigen, um von der Oberfläche eines 100 ml Wasser mit einer auf 20 °C stabil gehaltenen Temperatur enthaltenden Becherglases zu verschwinden, wenn dieses Pulver von angereichtertem Troxerutin auf die Oberfläche des Wassers im Becherglas gegossen wird, und mit einer Benetzbarkeit von unter 100 Sekunden, wenn die Benetzbarkeit in einem Versuch gemessen wird, der darin besteht, dass die Zeit gemessen wird, die dieses angereicherte Troxerutin benötigt, um durch in einem Behälter enthaltenes Wasser mit einer auf 20 °C gehaltenen Temperatur benetzt zu werden, wenn dieses angereicherte Troxerutin in Form von Karotten der Größe 2 mit einer Höhe von 3 mm und einem Gewicht von 63 ± 2 mg auf die Oberfläche des Wassers aufgelegt werden.

2. Angereichertes Troxerutin, **dadurch gekennzeichnet, dass** es zumindest 92 Gew.-% 7,3',4'-Trihydroxyethylrutosid, zwischen 2 und 4 Gew.-% 5,7,3',4'-Tetrahydroxyethylrutosid und zwischen 1 und 3 Gew.-% 7,4'-Dihydroxyethylrutosid umfasst.

3. Angereichertes Troxerutin nach Anspruch 1 oder 2, **dadurch gekennzeichnet, dass** der Gehalt an 7,3',4'-Trihydroxyethylrutosid 93 Gew.-% oder mehr beträgt.

4. Angereichertes Troxerutin nach einem der Ansprüche 2 und 3, **dadurch gekennzeichnet, dass** es zumindest 93 Gew.-% 7,3',4'-Trihydroxyethylrutosid, 2 bis 3,5 Gew.-% 5,7,3',4'-Tetrahydroxyethylrutosid und 1,7 bis 2,5 Gew.-% 7,4'-Dihydroxyethylrutosid umfasst.

5. Pharmazeutische Zusammensetzung, die angereichertes Troxerutin nach einem der Ansprüche 1 bis 4 zusammen mit einem pharmazeutischen Arzneimittelträger enthält, vorzugsweise zur oralen Verabreichung.

6. Pharmazeutische Zusammensetzung nach Anspruch 5 mit erhöhter Löslichkeit und hoher Auflösungsgeschwindigkeit auf der Basis von angereichertem Troxerutin zusammen mit einem für dessen orale Verabreichung geeigneten Arzneimittelträger mit einer Auflösungszeit von unter 140 Sekunden, wenn die Zeit in einem Versuch gemessen wird, der darin besteht, dass 7,25 g Körnchen in ein 200 ml Wasser enthaltendes 250-ml-Becherglas (Kragendurchmesser 78 mm, Höhe 95 mm) gegossen werden (Temperatur im Innenraum um die 20 °C, Rühren mit einem Magnetrührstäbchen mit den Maßen 35 x 6,5 mm, Geschwindigkeit 4 des Geräts (IKAMAG)) und die Zeit für das Auflösen der Körnchen gemessen wird.

7. Zusammensetzung nach Anspruch 6, **dadurch gekennzeichnet, dass** der Wirkstoff mit einem Anteil an 7,3',4'-Trihydroxyethylrutosid aus angereichertem Troxerutin besteht, worin der 7,3',4'-Trihydroxyethylrutosid-Anteil zumindest 92 Gew.-% beträgt.

8. Zusammensetzung nach Anspruch 7, **dadurch gekennzeichnet, dass** das angereicherte Troxerutin eine Zusammensetzung aufweist, die jener in einem der Ansprüche 2 bis 4 entspricht.

9. Verwendung von angereichertem Troxerutin nach einem der Ansprüche 1 bis 4 zur Herstellung eines Medikaments zur Behandlung von mit venolymphatischer Insuffizienz im Zusammenhang stehenden Symptomen (schwere Beine, Schmerzen, anfängliche Unruhe der Beine beim Hinlegen) und zur Behandlung von Anzeichen des Auftretens von Hämorrhoiden.

10. Verfahren zur Herstellung von Troxerutin, das zumindest 92 Gew.-% 7,3',4'-Trihydroxyethylrutosid, zwischen 2 und 4 Gew.-% 5,7,3',4'-Tetrahydroxyethylrutosid und zwischen 1 und 3 Gew.-% 7,4'-Dihydroxyethylrutosid umfasst, das darin besteht, dass Rutin unter Wärmezufuhr mit einem Überschuss an Ethylenoxid in Gegenwart einer Base in Wasser umgesetzt wird, woraufhin Kristallisation in einem Alkohol bewirkt wird, **dadurch gekennzeichnet, dass** das Reaktionsmedium so eingeengt wird, dass im Kristallisationsmedium ein Wassergehalt zwischen 1 und 8 % erzielt wird.

11. Verfahren nach Anspruch 10, **dadurch gekennzeichnet, dass** der Wassergehalt zwischen 1 % und 6 % beträgt.

12. Verfahren nach einem der Ansprüche 10 und 11, **dadurch gekennzeichnet, dass** das Kristallisationslösungsmittel Methanol oder Isopropanol - entweder allein oder als Gemisch - ist.

13. Verfahren nach einem der Ansprüche 10 bis 12, **dadurch gekennzeichnet, dass** die Kristallisation 1 bis 2 Stunden lang mit einer Geschwindigkeit der Temperaturabnahme von über etwa 20 °C pro Stunde durchgeführt wird.

14. Verfahren nach einem der Ansprüche 10 bis 13, **dadurch gekennzeichnet, dass** die Kristallisation bei einer Temperatur zwischen 35 und 15 °C erfolgt.

## Claims

1. An enriched troxerutin with a content of at least 92% by weight of 7,3',4'-trihydroxyethylrutoside, between 2% and 4% by weight of 5,7,3',4'-tetrahydroxyethylrutoside, and between 1% and 3% by weight of 7,4'-dihydroxyethylrutoside, and a wettability, expressed in minutes, of less than 10 minutes, when this wettability is measured in a test consisting in measuring the time taken for 3.5 g of a powder of this enriched troxerutin to leave the surface of a beaker containing 100 ml of water, at a stabilised temperature of 20°C, when this enriched troxerutin powder is poured onto the surface of the water in this beaker, and having a wettability of less than 100 seconds on average when this wettability is measured in a test consisting in measuring the time taken for this enriched troxerutin to be wetted with the water contained in a container, such as a beaker, when this enriched troxerutin has been placed on the surface of the water, in the form of piles of size 2 and 3 mm in height and weighing 63 ± 2 mg, at a stabilised temperature of 20°C.

2. An enriched troxerutin, **characterized in that** it comprises at least 92% by weight of 7,3',4'-trihydroxyethylrutoside, between 2% and 4% by weight of 5,7,3',4'-tetrahydroxyethylrutoside and between 1% and 3% by weight of 7,4'-dihydroxyethylrutoside.

3. The enriched troxerutin according to claim 1 or 2, **characterized in that** its content of 7,3',4'-trihydroxyethylrutoside is greater than or equal to 93% by weight.

4. The enriched troxerutin according to any one of claims 2 and 3, **characterized in that** it comprises at least 93% of 7,3',4'-trihydroxyethylrutoside, 2% to 3.5% of 5,7,3',4'-tetrahydroxyethylrutoside and from 1.7% to 2.5% of 7,4'-dihydroxyethylrutoside.

5. A pharmaceutical composition containing an enriched troxerutin according to any of claims 1 to 4, in combination with a pharmaceutical excipient, preferably for oral administration.

6. A pharmaceutical composition according to claim 5, with high solubility and a high dissolution speed, based on enriched troxerutin combined with an excipient which is suitable for its oral administration having a dissolution time of less than 140 seconds on average when this time is measured in a test consisting in pouring 7.25 g of grains into a 250 ml beaker (neck diameter 78 mm, height 95 mm) which contains 200 ml of water (room temperature at about 20°C, stirring with a 35 × 6.5 mm magnetic bar, machine speed 4 (IKAMAG) and in measuring the dissolution time of the grains.

7. A composition according to claim 6, **characterized in that** the active principle, of which 7,3',4'-trihydroxyethylrutoside forms a part, consists of an enriched troxerutin in which the 7,3',4'-trihydroxyethylrutoside is in an amount of at least 92% by weight.

8. A composition according to claim 7, **characterized in that** the enriched troxerutin has a constitution in accordance with that of any one of claims 2 to 4.

9. The use of an enriched troxerutin according to any one of claims 1 to 4, for the production of a medicament for treating symptoms in relation with venolymphatic insufficiencies (heavy legs, pain, primo-decubitus restless legs) and the treatment of functional signs associated with an attack of haemorrhoids.

10. A process for preparing troxerutin comprising at least 92% by weight of 7,3',4'-trihydroxyethylrutoside, between 2% and 4% by weight of 5,7,3',4'-tetrahydroxyethylrutoside, and between 1% and 3% by weight of 7,4'-dihydroxyethylrutoside, which consists in reacting rutin, under hot conditions, with an excess of ethylene oxide in water in the presence of a base, and then in bringing about a crystallization in an alcohol, **characterized in that** the reaction medium is concentrated so as to obtain, in the crystallization medium, a water content between 1% and 8%.

11. The process according to claim 10, **characterized in that** the water content is between 1% and 6%.

12. The process according to any one of claims 12 and 13, **characterized in that** the crystallization solvent is methanol or isopropanol, alone or as a mixture.

13. The process according to any one of claims 10 to 12, **characterized in that** the crystallization is carried out with a temperature descent speed of greater than 20°C per hour over 1 to 2 hours.

14. The process according to any one of claims 10 to 13, **characterized in that** the crystallization is carried out at a temperature of between 35°C and 15°C.
